(19) Europäisches Patentamt
European Patent Office
Office européen des brevets

(11) Veröffentlichungsnummer: **0 008 458 A1**

(12) # EUROPÄISCHE PATENTANMELDUNG

(21) Anmeldenummer: 79103077.8

(22) Anmeldetag: 22.08.79

(51) Int. Cl.³: C 07 D 249/08
C 07 D 409/06, C 07 D 405/06
C 07 D 401/06, A 01 N 43/64

(30) Priorität: 24.08.78 DE 2836945

(43) Veröffentlichungstag der Anmeldung: 05.03.80 Patentblatt 80/5

(84) Benannte Vertragsstaaten: AT BE CH DE FR GB IT NL SE

(71) Anmelder: HOECHST AKTIENGESELLSCHAFT
Zentrale Patentabteilung Postfach 80 03 20
D-6230 Frankfurt/Main 80(DE)

(72) Erfinder: Mildenberger, Hilmar, Dr.
Fasanenstrasse 24
D-6233 Kelkheim (Taunus)(DE)

(72) Erfinder: Maier, Thomas, Dr.
Rauenthaler Weg 22
D-6000 Frankfurt am Main 71(DE)

(72) Erfinder: Sachse, Burkhard, Dr.
Parkstrasse 18
D-6233 Kelkheim (Taunus)(DE)

(54) **Derivate des 1,2,4-Triazols, Verfahren zu ihrer Herstellung und diese enthaltende Schädlingsbekämpfungsmittel.**

(57) Gegenstand der Erfindung sind neue 1, 2, 4-Triazolderivate der Formel I,

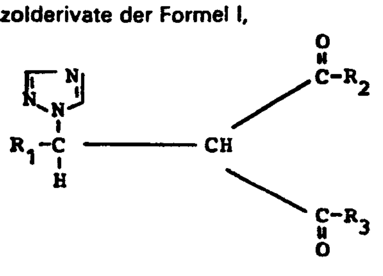

(I)

in der

$R_1$ gegebenenfalls substituiertes $(C_1$-$C_{12})$-Alkyl, gegebenenfalls substituiertes $(C_2$-$C_5)$-Alkenyl, gegebenenfalls substituiertes Cycloalkyl, gegebenenfalls substituiertes Cycloalkenyl, gegebenenfalls substituiertes Phenyl, Furanyl, Thienyl, Pyridyl oder $(C_1$-$C_4)$-Alkoxycarbonyl, und

$R_2$, $R_3$, die gleich oder verschieden sein können, gegebenenfalls substituiertes $(C_1$-$C_{12})$-Alkyl, gegebenenfalls substituiertes Cycloalkyl, gegebenenfalls substituiertes Phenyl, gegebenenfalls substituiertes $(C_1$-$C_{12})$-Alkoxy, gegebenenfalls substituiertes $(C_5$-$C_6)$-Cycloalkoxy, $(C_3$-$C_4)$-Alkenyloxy, Halogen-$(C_3$-$C_4)$-alkenyloxy, $(C_5$-$C_6)$-Cycloalkenyloxy, gegebenenfalls substituiertes $(C_3$-$C_4)$-Alkinyloxy, gegebenenfalls substituiertes Phenoxy, $(C_1$-$C_4)$-Alkylamino, Di-$(C_1$-$C_4)$-alkylamino oder gegebenenfalls substituiertes Anilino bedeuten, ferner ein Verfahren zu deren Herstellung sowie deren Verwendung als Schädlingsbekämpfungsmittel.

EP 0 008 458 A1

HOECHST AKTIENGESELLSCHAFT HOE 78/F 173 Dr.GM/E

**Derivate des 1,2,4-Triazols, Verfahren zu ihrer Herstellung und diese enthaltende Schädlingsbekämpfungsmittel**

Gegenstand der vorliegenden Erfindung sind neue 1, 2, 4-Triazolderivate, ihre Herstellung und Verwendung als Schädlingsbekämpfungsmittel insbesondere im Pflanzenschutz.

Die erfindungsgemäßen Verbindungen besitzen die allgemeine Formel I,

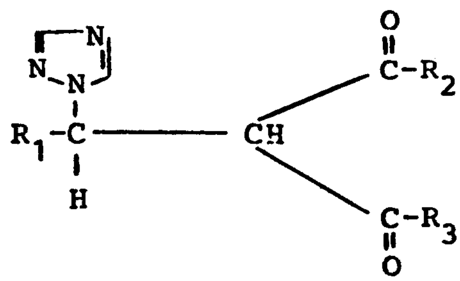

(I)

worin

$R_1$ $(C_1-C_{12})$-Alkyl, das gegebenenfalls zusätzlich vorzugsweise durch Halogen, Phenyl, das ein- bis zweifach durch Halogen, $(C_1-C_4)$-Alkoxy oder $(C_1-C_4)$-Alkyl substituiert sein kann, $(C_1-C_4)$-Alkoxy oder Phenoxy, welches ein- bis zweifach durch Halogen, $(C_1-C_4)$-Alkoxy oder $(C_1-C_4)$-Alkyl substituiert sein kann, substituiert ist, $(C_2-C_5)$-Alkenyl, das gegebenenfalls zusätzlich durch Phenyl, das ein- bis zweifach durch Halogen oder $(C_1-C_4)$-Alkoxy substituiert sein kann, substituiert ist, Cycloalkyl, vorzugsweise mit 5 oder 6 C-Atomen, oder substituiertes Cycloalkyl mit vorzugsweise insgesamt 5 bis 9 C-Atomen, insbesondere durch Alkylreste substituiertes Cycloalkyl, Cycloalkenyl, vorzugsweise mit 5 oder 6 C-Atomen, oder substituiertes Cycloalkenyl mit vorzugsweise insgesamt 5 bis 9 C-Atomen, insbesondere durch Alkylreste substituiertes Cycloalkenyl, Phenyl oder substituiertes Phenyl, das insbesondere ein- bis dreifach durch Alkyl, vorzugsweise $(C_1-C_{12})$-

Alkyl, Halogen, $(C_1-C_5)$-Alkoxy, Hydroxy, Nitro, Dialkylamino mit vorzugsweise $(C_1-C_6)$-Alkylgruppen, oder den Methylendioxy-Rest substituiert ist, weiterhin Furanyl, Thienyl, Pyridyl oder $(C_1-C_4)$-Alkoxycarbonyl, und

$R_2$, $R_3$, die gleich oder verschieden sein können, $(C_1-C_{12})$-Alkyl, das gegebenenfalls zusätzlich vorzugsweise durch Halogen, insbesondere ein- oder zweifach, und/oder ein- oder zweifach durch $(C_1-C_6)$-Alkoxy, ferner durch $(C_1-C_4)$-Alkylthio, Phenylthio, welches ein- oder zweifach durch Halogen oder $(C_1-C_4)$-Alkyl substituiert sein kann, $(C_1-C_4)$-Alkylamino, Di-$(C_1-C_4)$-alkylamino, Phenyl oder Phenoxy, welches ein- oder zweifach durch Halogen, $(C_1-C_4)$-Alkyl oder $(C_1-C_3)$-Alkoxy substituiert sein kann, substituiert ist, oder Cycloalkyl, vorzugsweise mit 5 oder 6 C-Atomen, oder substituiertes Cycloalkyl mit vorzugsweise insgesamt 5 bis 9 C-Atomen, insbesondere durch Alkylreste substituiertes Cycloalkyl, Phenyl oder substituiertes Phenyl, das insbesondere ein- bis dreifach durch Alkyl, vorzugsweise $(C_1-C_{12})$-Alkyl, Halogen, $(C_1-C_5)$-Alkoxy oder Hydroxy substituiert ist, $(C_1-C_{12})$-Alkoxy, das gegebenenfalls zusätzlich durch 1 bis 6, vorzugsweise 1 oder 2 Halogenatome und/oder $(C_1-C_6)$-Alkoxy, $(C_1-C_4)$-Alkylthio, $(C_1-C_6)$-Alkoxy-$(C_2-C_6)$-alkoxy, Halogen-$(C_1-C_2)$-alkoxy, Methoxy-äthoxy-äthoxy, $(C_1-C_4)$-Alkylamino, Di-$(C_1-C_4)$-alkylamino, Phenyl, das ein- oder zweifach durch Halogen, $(C_1-C_4)$-Alkyl, $(C_1-C_3)$-Alkoxy oder N-$(C_1-C_4)$-Alkyl-anilino substituiert sein kann, Oxiranyl oder Phenoxy, das ein- oder zweifach durch Halogen oder $(C_1-C_4)$-Alkyl substituiert sein kann, substituiert ist, oder $(C_5-C_6)$-Cycloalkoxy, das gegebenenfalls durch Halogen oder Methyl substituiert ist, $(C_3-C_6)$-Alkenyloxy, Halogen-$(C_3-C_6)$-alkenyloxy, $(C_5-C_6)$-Cycloalkenyloxy, $(C_3-C_4)$-Alkinyloxy, das gegebenenfalls ein- oder zweifach

durch $C_1$-$C_6$)-Alkyl, Phenyl, Halogen oder ($C_1$-$C_2$)-Alkoxy substituiert ist, oder Phenoxy, das gegebenenfalls ein- bis dreifach durch ($C_1$-$C_4$)-Alkyl, ($C_1$-$C_4$)-Alkoxy, Halogen, Nitro oder Trifluormethyl substituiert ist, oder ($C_1$-$C_4$)-Alkylamino, Di-($C_1$-$C_4$)-alkylamino, Anilino, das gegebenenfalls am Benzolring ein- oder zweifach durch ($C_1$-$C_4$)-Alkyl, Halogen, ($C_1$-$C_3$)-Alkoxy oder am Anilin-N-atom durch eine ($C_1$-$C_4$)-Alkylgruppe substituiert ist, bedeuten.

Bevorzugte Reste $R_1$ sind z. B. ($C_1$-$C_{12}$)-Alkyl, insbesondere ($C_1$-$C_6$)-Alkyl, Cycloalkyl mit 5 oder 6 C-Atomen, alkylsubstituiertes Cycloalkyl mit insgesamt 5 bis 9 C-Atomen, Cycloalkenyl mit 5 oder 6 C-Atomen, alkylsubstituiertes Cycloalkenyl mit insgesamt 5 bis 9 C-Atomen, Phenyl, substituiertes Phenyl, insbesondere ein- bis dreifach durch ($C_1$-$C_{12}$)-Alkyl, Halogen, ($C_1$-$C_5$)-Alkoxy, Hydroxy, Nitro, Di-($C_1$-$C_6$)-alkylamino oder den Methylendioxy-Rest substituiertes Phenyl, Furanyl, Thienyl oder Pyridyl.
Besonders bevorzugte Reste $R_1$ sind z. B. Phenyl, Chlorphenyl und Methoxyphenyl.

Bevorzugte Reste $R_2$, $R_3$ sind z. B. ($C_1$-$C_{12}$)-Alkyl, gegebenenfalls substituiertes ($C_1$-$C_{12}$)-Alkoxy, Cycloalkyl mit 5 oder 6 C-Atomen, alkylsubstituiertes Cycloalkyl mit insgesamt 5 bis 9 C-Atomen, Phenyl, substituiertes Phenyl, insbesondere ein- bis dreifach durch ($C_1$-$C_{12}$)-Alkyl, Halogen, ($C_1$-$C_5$)-Alkoxy oder Hydroxy substituiertes Phenyl, ($C_5$-$C_6$)-Cycloalkoxy, Benzyloxy oder Diäthoxymethyl.

Besonders bevorzugte Reste $R_2$, $R_3$ sind z. B. ($C_1$-$C_6$)-Alkyl, insbesondere Methyl, Äthyl, Propyl, Butyl, Phenyl, Chlorphenyl, Methoxy, Äthoxy, Propyloxy, Butyloxy, Oktyloxy.

Die in den Resten $R_1$, $R_2$, $R_3$ aufgeführten Alkylreste können sowohl geradkettig als auch verzweigt sein. Halogen steht vorzugsweise für Fluor, Chlor, Brom.

Die Verbindungen der allgemeinen Formel I erhält man, indem man Verbindungen der allgemeinen Formel II,

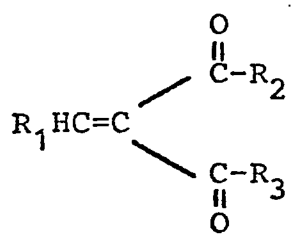

(II)

worin $R_1$, $R_2$ und $R_3$ die Bedeutungen wie in Formel I haben, mit 1,2,4-Triazol umsetzt.

Die Reaktionskomponenten werden im allgemeinen in stöchiometrischen Mengen eingesetzt. Auch ein Überschuß an einer der Reaktionskomponenten ist zwar prinzipiell möglich, bringt aber keine Vorteile.

Die Verbindungen der Formel II sind bereits bekannt und lassen sich z.B. durch Knoevenagel-Kondensation darstellen.

Die Addition von 1,2,4-Triazol an die Verbindungen der Formel II wird je nach Löslichkeit der Ausgangskomponenten entweder ohne oder vorteilhafter in einem unter den Reaktionsbedingungen inerten Lösungsmittel durchgeführt. Geeignete Lösungsmittel sind z.B. Chloroform, $(C_1-C_4)$-Alkohole, Triäthylamin, Methylenchlorid, Aceton, Acetonitril. Die Lösungsmittelmenge kann von Bruchteilen der Menge des Reaktantengemisches bis zu deren gleicher Menge oder ggf. deren Vielfachem betragen. Auch Mischungen aus verschiedenen Lösungsmitteln können eingesetzt werden.

Obwohl die Umsetzung im allgemeinen bereits beim Vermischen der Reaktionskomponenten unter Bildung der Verbindungen der Formel I einsetzt, ist es zweckmäßig und vorteilhaft, dem Reaktionsgemisch noch einen Katalysator zuzusetzen. Als Katalysatoren geeignet sind anorganische oder organische basische Verbindungen, insbesondere tertiäre Amine. Die einzusetzende Menge an basischem Katalysator ist nicht kritisch und kann z.B. von $<0,1$ Gew.-% bis zu etwa 100 Gew.-%, bezogen auf die Reaktantengemischmenge, oder auch darüber betragen. Beispielsweise können tertiäre Amine, insbesondere Triäthylamin vorteilhaft gleichzeitig als Katalysator und als Lösungsmittel eingesetzt werden.

Die Additionsreaktion zwischen den Ausgangskomponenten erfolgt bisweilen schwach, bisweilen stärker exotherm. Die Durchführung und Vervollständigung der Reaktion richtet sich nach der Reaktionsfähigkeit der Verbindungen der Formel II und der Löslichkeit der Verbindungen der Formeln I und II.

So ist es z.B. bei homogenen Systemen vorteilhaft, die Umsetzung bei Raumtemperatur einzuleiten, die exotherme Reaktion abklingen zu lassen und den Ansatz anschließend bis zu 24 Stunden bei Raumtemperatur stehen zu lassen.

Bei Reaktionsgemischen, deren Bestandteile nach Beginn der Reaktion unvollständig löslich bleiben oder bei denen das Reaktionsprodukt frühzeitig auskristallisiert, ist es zweckmäßig, das Gemisch bis zu 2 Stunden auf Temperaturen von 50-90°C zu erhitzen.

Im übrigen ist die Reaktionstemperatur nicht kritisch. Sie wird vorzugsweise im Bereich zwischen etwa 0°C und 100°C gewählt, wobei auch Temperaturen unterhalb von 0°C möglich sind und die Temperaturobergrenze beispielsweise durch den Siedepunkt von ggf. mitverwendeten Lösungsmitteln bestimmt sein, aber auch höher als 100°C liegen kann. Zweckmäßig und vorteilhaft wird die Additionsreaktion im allgemeinen bei Raumtemperatur eingeleitet.

Die Isolierung und Reinigung der erfindungsgemäß erhaltenen Verbindungen der Formel I erfolgt nach üblichen Methoden.

In der Regel kristallisieren die Reaktionsprodukte bei Raumtemperatur aus dem Reaktionsgemisch aus. Ist dies nicht der Fall, so werden beispielsweise alle flüchtigen Bestandteile unter Vakuum bei 20-60°C aus dem Reaktionsgemisch abdestilliert und der Rückstand aus einem geeigneten Lösungsmittel umkristallisiert oder gewaschen und das Umsetzungsprodukt isoliert.

Die bei dem erfindungsgemäßen Verfahren resultierenden Ausbeuten an Verbindungen der Formel I können sehr hoch sein.

Die beanspruchten Verbindungen der Formel I zeichnen sich durch eine sehr gute fungizide Wirkung aus. Mit ihrer Hilfe lassen sich bereits in das pflanzliche Gewebe eingedrungene pilzliche Krankheitserreger kurativ bekämpfen. Dies ist besonders wichtig und vorteilhaft bei solchen Pilzkrankheiten, die nach eingetretener Infektion mit den sonst üblichen Fungiziden nicht mehr bekämpft werden können. Das Wirkungsspektrum der beanspruchten Verbindungen erfaßt neben Phytophthora infestans, Plasmopara viticola, Piricularia oryzae und Puccinia triticina vor allem Echte Mehltauarten im Obst-, Gemüse-, Getreide- und Zierpflanzenbau. Besonders hervorzuheben ist die ausgezeichnete Wirkung der Verbindungen gegen Benzimidazolcarbamatresistente Mehltauarten.

Für die Anwendung im Pflanzenschutz können die Verbindungen der Formel I in üblicher Weise als Stäube, Spritzpulver, Beizmittel, Dispersionen, Lösungen oder Emulsionskonzentrate formuliert werden. Der Gehalt an Wirkstoff der Formel I liegt in den fungiziden Mitteln im allgemeinen zwischen 2-95 Gew.-%, vorzugsweise bei 10-90 Gew.-%. Daneben enthalten die genannten Wirkstoff-Formulierungen

gegebenenfalls die jeweils üblichen Haft-, Netz-, Dispergier-, Emulgier-, Penetrations-, Lösungsmittel-, Füll- und Trägerstoffe.

Die beanspruchten Verbindungen der Formel I eignen sich auch für den Einsatz im technischen Bereich, beispielsweise in Holzschutzmitteln, auf dem Anstrichfarbensektor oder als Konservierungsmittel, z.B. in Kühlschmiermitteln für die Metallbearbeitung.

Die Erfindung wird durch folgende Beispiele näher erläutert:

## A. Herstellungsbeispiele

### Beispiel 1

2-Methoxycarbonyl-4-methyl-3-(1,2,4-triazol-1-yl)-valeriansäuremethylester

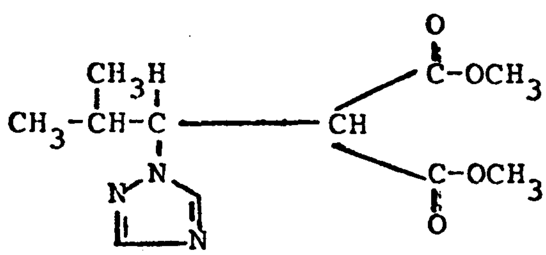

Ein Gemisch aus 55,8 g (0,3 Mol) Isobutylidenmalonsäuredimethylester, 20,7 g (0,3 Mol) 1,2,4-Triazol und 25 ml Triäthylamin erreicht nach wenigen Minuten eine Innentemperatur von 70°C, wobei ein weißer Niederschlag entsteht. Nach 30 Minuten wird 7 Stunden bei 90°C gerührt, der entstandene Feststoff zerkleinert, abgesaugt und mit Petroläther gewaschen. Man erhält 73,94 g (96,65 % d.Th.) 2-Methoxycarbonyl-4-methyl-3-(1,2,4-triazol-1-yl)-valeriansäuremethylester als weiße Kristalle vom Fp. 118°C.

Analyse: (M= 255,3) ber.: C 51,76 %; H 6,71 %; N 16,46 %
gef.: C 51,60 %; H 6,70 %; N 16,30 %.

Beispiel 2

Diäthyl-2-/(phenyl)-(1,2,4-triazol-1-yl)-methyl/-malonat

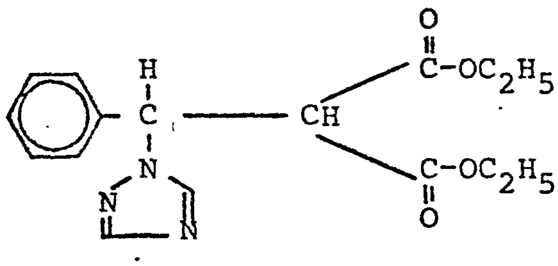

a) Ein Gemisch aus 74,4 g (0,3 Mol) Benzylidenmalonsäurediäthylester, 20,7 g (0,3 Mol) 1,2,4-Triazol, 40 ml Triäthylamin und 20 ml Äthanol wird 20 Stunden bei Raumtemperatur gerührt. Kristallisation tritt beim Reiben mit einem Glasstab ein. Die weißen Kristalle werden abgesaugt, mit Diisopropyläther gewaschen, getrocknet und man erhält 82 g vom Fp. 79-82°C. Die eingeengte Mutterlauge wird zur Kristallisation gebracht, mit Diisopropyläther versetzt, der Feststoff abgesaugt und man erhält weitere 8,2 g, Fp. 79°C. Gesamtausbeute: 90,2 g (94,85 % d.Th.) Diäthyl-2-/(phenyl)-(1,2,4-triazol-1-yl)-methyl/-malonat.

Analyse: $C_{16}H_{19}N_3O_4$ (M= 217,35)

ber.: C 60,6 %; H 6,0 %; N 13,2 %
gef.: C 60,6 %; H 6,2 %; N 13,3 %

b) Ein Gemisch aus 7,5 g (0,03 Mol) Benzylidenmalonsäurediäthylester, 2,1 g (0,03 Mol) 1,2,4-Triazol und 10 Tropfen Triäthylamin wird 3 Tage bei Raumtemperatur gerührt. Nach 2 Stunden Erhitzen auf 70°C und Abdestillieren der flüchtigen Bestandteile unter Wasserstrahlvakuum wird das verbleibende Öl durch Impfen zur Kristallisation gebracht, die Kristallmasse zerkleinert, mit Diisopropyläther gewaschen und abgesaugt. Man erhält 5,56 g weiße Kristalle vom Fp. 80-81°C. Die eingeengte Mutterlauge wird erneut mit Diisopropyläther behandelt. Man erhält weitere 1,75 g. Gesamtausbeute: 7,31 g (76,9 % d. Th.) Diäthyl-2-/(phenyl)-(1,2,4-triazol-1-yl)-methyl/-malonat.

c) Ohne basischen Katalysator
Durchführung des Versuchs wie unter b) beschrieben, jedoch ohne Erhitzen.
Ausbeute: 6,53 g (68,7 % d.Th.) Diäthyl-2-/(phenyl)-1,2,4-triazol-1-yl)-methyl/-malonat vom Fp. 76°C.

Beispiel 3

Äthyl-2-/(phenyl)-(1,2,4-triazol-1-yl)-methyl/-3-oxo-butanoat

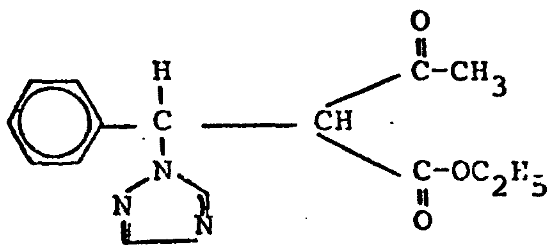

Zu 65,4 g (0,3 Mol) Benzylidenacetessigsäureäthylester in 25 ml Triäthylamin gibt man bei Raumtemperatur (21°C) 20,7 g (0,3 Mol) 1,2,4-Triazol. Die Innentemperatur des Reaktionsgemisches steigt in 30 Minuten um ca. 10°C. Nach 1 Stunde erstarrt das Reaktionsgemisch schlagartig, wobei die Innentemperatur bis auf 60°C ansteigt. Der resultierende weiße Feststoff wird mit Diisopropyläther gewaschen und man erhält 79,6 g (92,5 % d.Th.) Äthyl-2-/(phenyl)-(1,2,4-triazol-1-yl)-methyl/3-oxo-butanoat vom Fp. 88-90°C. Einengen der Mutterlauge ergibt 5,5 g unreines Produkt vom Schmp. 66-69°C, auf dessen Reinigung verzichtet wurde.

Analyse: $C_{15}H_{17}N_3O_3$ (M= 287,3)

| | | | |
|---|---|---|---|
| ber.: | C 62,7 %; | H 6,0 %; | N 14,6 % |
| gef.: | C 62,6 %; | H 5,9 %; | N 14,6 % |

Beispiel 4

3-/(Phenyl)-(1,2,4-triazol-1-yl)-methyl/-pentan-2,4-dion

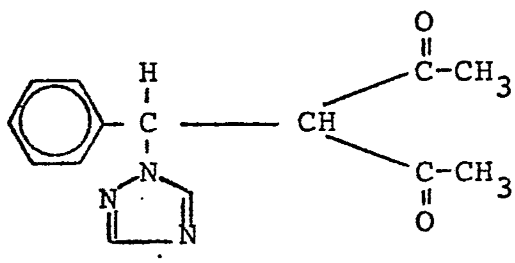

Zu 18,8 g (0,1 Mol) Benzylidenacetylaceton in 16 ml Triäthylamin gibt man 6,9 g (0,1 Mol) 1,2,4-Triazol. Die Innentemperatur steigt in 30 Minuten um 10°C; dabei bilden sich 2 Phasen. Man erhitzt wenige Minuten auf 50°C, läßt 12 Stunden stehen, wobei das Reaktionsprodukt auskristallisiert, saugt ab, wäscht mit Diisopropyläther und erhält 22,8 g (88,8 % d.Th.) 3-/(Phenyl)-1,2,4-triazol-1-yl)-methyl/-pentan-2,4-dion, Fp. 90-92°C.

Analyse: $C_{14}H_{15}N_3O_2$ (M= 257,3)

ber.: C 65,4 %; H 5,9 %; N 16,3 %
gef.: C 65,5 %; H 6,0 %; N 16,3 %

Beispiel 5

Diäthyl-2-/4-methoxy-phenyl)-(1,2,4-triazol-1-yl)methyl/-malonat

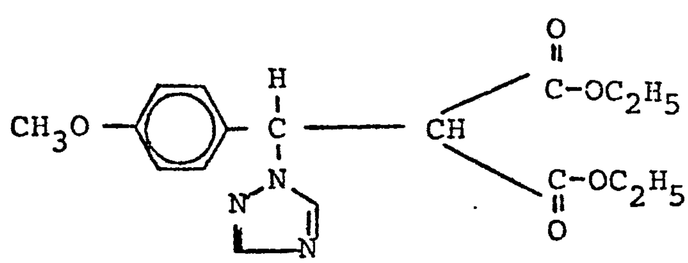

83,4 g (0,3 Mol) 4-Methoxybenzylidenmalonsäurediethylester und 20,7 g (0,3 Mol) 1,2,4-Triazol werden in 40 ml Triäthylamin und 20 ml Äthanol bei Raumtemperatur gerührt, wobei allmählich alle Komponenten in Lösung gehen. Nach anfangs schwacher positiver Wärmetönung von ca. 2°C erstarrt das Reaktionsgemisch innerhalb einer Stunde, wobei die Innentemperatur auf 41°C steigt. Man läßt 15 Stunden stehen, erhitzt dann noch 10 Minuten auf 70°C und läßt langsam auskristallisieren. Nach Absaugen und Waschen mit Diisopropyläther erhält man 98,0 g (94,2 % d.Th.) Diäthyl-2-/(4-methoxy-phenyl)-(1,2,4-triazol-1-yl)-methyl/-malonat als weiße Kristalle vom Fp. 98-101°C.

Analyse: $C_{17}H_{21}N_3O_5$ (M= 347,4)

| | | | |
|---|---|---|---|
| ber.: | C 58,8 %; | H 6,1 %; | N 12,1 % |
| gef.: | C 58,3 %; | H 6,1 %; | N 12,5 % |

Beispiel 6

Dimethyl-2-/(thien-2-yl)-(1,2,4-triazol-1-yl)-methyl/-malonat

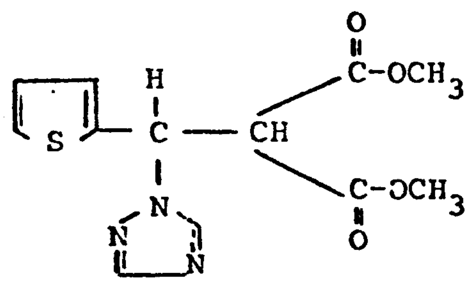

Ein Gemisch aus 90,4 g (0,4 Mol) Thienylidenmalonsäuredimethylester, 27,6 (0,4 Mol) 1,2,4-Triazol, 40 ml Triäthylamin und 200 ml Mehtanol zeigt keine exotherme Reaktion. Nach kurzem Erwärmen auf 50°C und Abkühlen tritt Kristallisation ein. Nach Absaugen und Waschen mit Diisopropyläther erhält man 77,0 g weiße Kristalle vom Schmelzpunkt 125 - 127°C. Die Mutterlauge wird weitgehend eingeengt, die erhaltene Kristallmasse wie oben behandelt. Man erhält weitere 40,7 g vom Fp. 122 - 124°C.

Gesamtausbeute: 117,7 g (99,7 % d.Th.) Dimethyl-2-/¯(thien-2-yl)-(1,2,4-triazol-1-yl)-methyl_7-malonat.

| | | | | | |
|---|---|---|---|---|---|
| Analyse: | $C_{12}H_{13}N_3SO_4$ | | (M= 295,3) | | |
| ber.: | C 48,8 %; | H 4,4 %; | N 14,2 %; | S 10,9 % | |
| gef.: | C 48,8 %; | H 4,4 %; | N 14,4 %; | S 10,9 % | |

<u>Beispiele 7 - 126</u>

In der Tabelle 1 sind die Reste $R_1$, $R_2$ und $R_3$ in Formel I der nach den Beispielen 7 bis 126 aus den entsprechenden Verbindungen der Formel II und 1,2,4-Triazol hergestellten Verbindungen sowie deren Schmelzpunkte aufgeführt. Im einzelnen wurde bei deren Herstellung wie folgt verfahren:

Analog Beispiel 1 wurden die Beispiele 7, 8, 11 und 12, jedoch ohne Erhitzen durchgeführt; ferner Beispiel 27, bei dem lediglich 10 Minuten auf 50°C erhitzt wurde; weiterhin Beispiele 63, 64 und 66, 70, 71, 78, 80-83, 90, 95-99, 109, 110, 111, 115, 116, 123 und 125, bei denen 15 Minuten auf 70°C erhitzt wurde. Blieb die Kristallisation des Reaktionsproduktes aus, so wurde das Reaktionsgemisch zunächst am Rotationsverdampfer unter reduziertem Druck eingeengt und das verbleibende Öl gegebenenfalls zur Kristallisation gebracht; ferner Beispiel 42, das unter Zusatz von Methylenchlorid auf die Rückflußtemperatur von 42°C erhitzt wurde.

Analog Beispiel 2a wurden die Beispiele 21, 23, 31, 32 und 50 durchgeführt, wobei bei den Nicht-Äthylesterreaktanten anstelle von Äthanol jeweils der mit der Esterkomponente korrespondierende Alkohol als Lösungsmittelkomponente eingesetzt wurde.

Analog Beispiel 3 wurden die Beispiele 9, 13 bis 20, 24, 33, 34, 48 durchgeführt; ferner die Beispiele 35, 44, 65, 73, 74, 75, 84, 85, 93, 94, 100-108, 112-114, 117-122, 124 und 126, die jedoch 10 Minuten auf 75°C erhitzt wurden; ferner die Beispiele 29, 38, 40, 43, 45, 60, 67, 72, 76 und 86, bei denen bezüglich Lösungsmittel und Temperatur außerdem analog

Beispiel 5 gearbeitet wurde; ferner Beispiel 17, das unter Zusatz von Methanol durchgeführt wurde.

Analog Beispiel 4 wurden die Beispiele 10, 25, 30, 36, 46, 49, 88, 89, 91 und 92 durchgeführt; ferner Beispiel 22, aber unter Zusatz von Chloroform als Lösungsmittel.

Analog den Beispielen 5 und 6, wobei bei den Nicht-Äthylesterreaktanten anstelle von Äthanol jeweils der mit der Esterkomponente korrespondierende Alkohol als Lösungsmittelkomponente eingesetzt wurde, wurden die Beispiele 26, 28, 37, 39, 41, 42, 47, 51, 54, 56-59, 61, 62, 68, 69, 77, 79 und 87 durchgeführt; ferner die Beispiele 52, 53 und 55, die jedoch 2 1/2 Stunden am Rückfluß gekocht wurden.

<u>Tabelle 1</u>

Formel I:

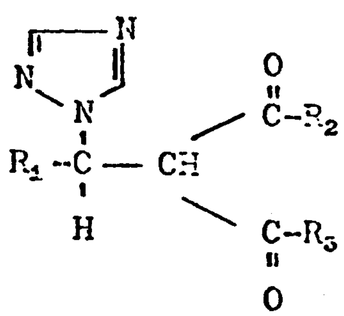

(I)

| Beispiel-Nr. | $R_1$ | $R_2$ | $R_3$ | Fp. °C |
|---|---|---|---|---|
| 7 | $CH_3CH_2CH_2-$ | $CH_3O-$ | $CH_3O-$ | 75-78 |
| 8 | $n-C_5H_{11}-$ | $CH_3O-$ | $CH_3O-$ | Oel |
| 9 | $n-C_5H_{11}-$ | $CH_3-$ | $C_2H_5O-$ | Oel |
| 10 | $n-C_5H_{11}-$ | $CH_3-$ | $CH_3-$ | 66-68 |

| Beispiel Nr. | $R_1$ | $R_2$ | $R_3$ | Fp. °C |
|---|---|---|---|---|
| 11 | 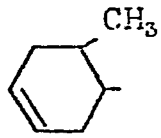 | $CH_3O-$ | $CH_3O-$ | 104 - 106 |
| 12 | 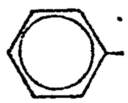 | $CH_3O-$ | $CH_3O-$ | 134 - 136 |
| 13 | 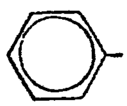 | $CH_3-$ | $CH_2CH_2CH_3O-$ | - |
| 14 | 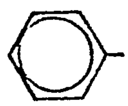 | $CH_3-$ | $n-C_4H_9O-$ | - |
| 15 | 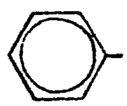 | $CH_3-$ | $iso-C_4H_9O-$ | $n_D^{20}=1.5207$ |
| 16 | 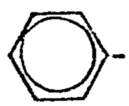 | $CH_3-$ | $(CH_3)_3CO-$ | 104 - 106 |
| 17 | 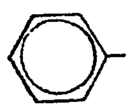 | $CH_3-$ | 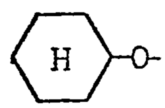 | 79 - 84 |
| 18 | 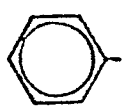 | $CH_3-$ | $n-C_8H_{17}O-$ | $n_D^{24}=1.5082$ |
| 19 | 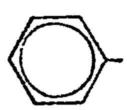 | $CH_3-$ | $n-C_{12}H_{25}O-$ | - |
| 20 | 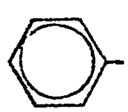 | $CH_3-$ | 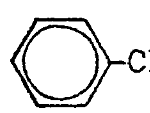 $-CH_2-O-$ | 97 - 98 |
| 21 | 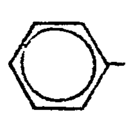 | 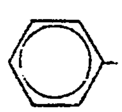 | $C_2H_5O-$ | 114 - 118 |

| Beispiel Nr. | $R_1$ | $R_2$ | $R_3$ | Fp. °C |
|---|---|---|---|---|
| 22 | 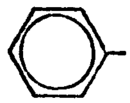 | 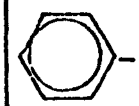 | $CH_3$- | 114 - 117 |
| 23 | $CH_3$- 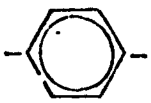 | $CH_3O$- | $CH_3O$- | 157 |
| 24 | $CH_3$- 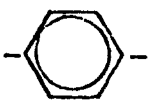 | $CH_3$- | $C_2H_5O$- | 81 - 84 |
| 25 | $CH_3$- 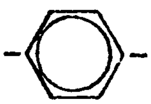 | $CH_3$- | $CH_3$- | 91 - 93 |
| 26 | 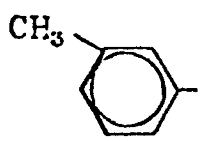 | $CH_3O$- | $CH_3O$- | 126 - 128 |
| 27 | 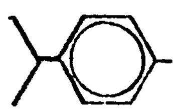 | $CH_3O$- | $CH_3O$- | 79 - 82 |
| 28 | 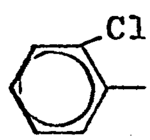 | $CH_3O$- | $CH_3O$- | 117 - 119 |
| 29 | 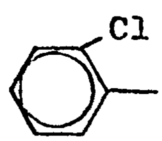 | $CH_3$- | $C_2H_5O$- | Semi-kristallin |
| 30 | 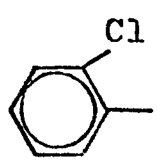 | $CH_3$- | $CH_3$- | 70 - 74 |

| Beispiel Nr. | $R_1$ | $R_2$ | $R_3$ | Fp. °C |
|---|---|---|---|---|
| 31 | Cl- 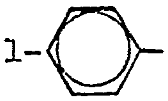 | $CH_3O-$ | $CH_3O-$ | 144 - 146 |
| 32 | Cl- 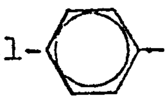 | $C_2H_5O-$ | $C_2H_5O-$ | 104 - 109 |
| 33 | Cl- 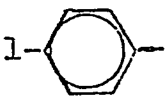 | $CH_3-$ | $C_2H_5O-$ | 86 - 90 |
| 34 | Cl- 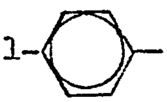 | $CH_3-$ | $(CH_3)_3CO-$ | 108 - 114 |
| 35 | Cl- 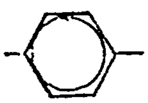 | 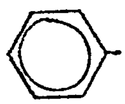 | $C_2H_5O-$ | 116 - 120 |
| 36 | Cl- 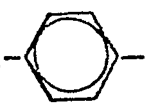 | $CH_3-$ | $CH_3-$ | 103 - 111 |
| 37 | 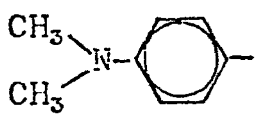 | $CH_3O-$ | $CH_3O-$ | 93 - 95 |
| 38 | 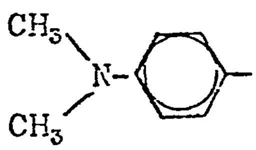 | $CH_3-$ | $C_2H_5O-$ | 105 - 109 |
| 39 | 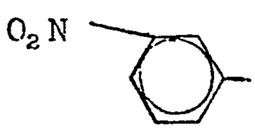 | $CH_3O-$ | $CH_3O-$ | 150 - 152 |
| 40 | 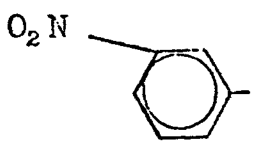 | $CH_3-$ | $C_2H_5O-$ | 88 - 93 |
| 41 | Cl- 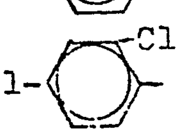 | $CH_3O-$ | $CH_3O-$ | 82 - 86 |

| Beispiel Nr. | $R_1$ | $R_2$ | $R_3$ | Fp. °C |
|---|---|---|---|---|
| 42 | 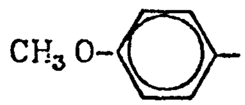 | $CH_3O-$ | $CH_3O-$ | 127 - 129 |
| 43 | 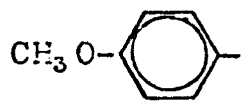 | $CH_3-$ | $C_2H_5O-$ | Semi-kristallin |
| 44 | 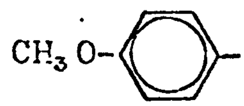 | $CH_3-$ | $(CH_3)_3CO-$ | 98 - 108 |
| 45 | 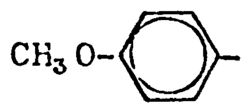 | 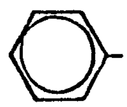 | $C_2H_5O-$ | 80 - 96 |
| 46 | 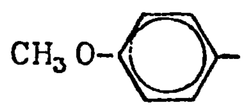 | $CH_3-$ | $CH_3-$ | 99 - 101 |
| 47 | 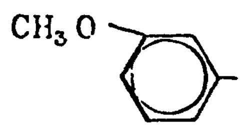 | $CH_3O-$ | $CH_3O-$ | 110 - 112 |
| 48 | 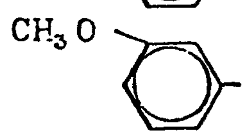 | $CH_3-$ | $C_2H_5O-$ | 77 - 83 |
| 49 | 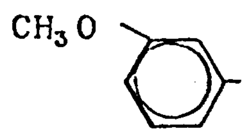 | $CH_3-$ | $CH_3-$ | 97 - 100 |
| 50 | 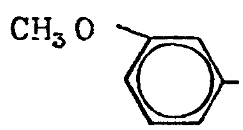 | $C_2H_5O-$ | $C_2H_5O-$ | 58 - 60 |
| 51 | 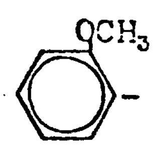 | $CH_3-$ | $C_2H_5O-$ | 82 - 84 |
| 52 | 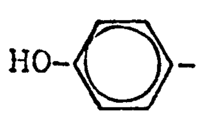 | $CH_3O-$ | $CH_3O-$ | 150 - 154 |
| 53 | 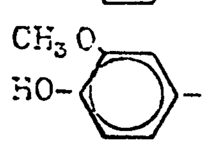 | $CH_3O-$ | $CH_3O-$ | 118 - 121 |

| Beispiel Nr. | $R_1$ | $R_2$ | $R_3$ | Fp. °C |
|---|---|---|---|---|
| 54 | 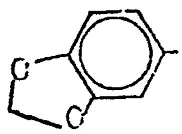 | $CH_3O-$ | $CH_3O-$ | 152 - 155 |
| 55 | 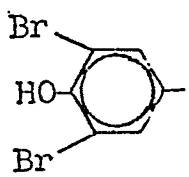 | $CH_3O-$ | $CH_3O-$ | – |
| 56 | 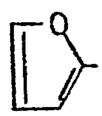 | $CH_3O-$ | $CH_3O-$ | 93 - 96 |
| 57 | 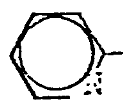 | $CH_3O-$ | $CH_3O-$ | 129 - 131 |
| 58 | 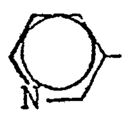 | $CH_3O-$ | $CH_3O-$ | 127 - 128 |
| 59 | 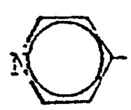 | $CH_3O-$ | $CH_3O-$ | 133 - 135 |
| 60 | 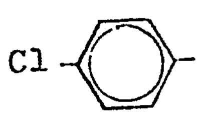 | 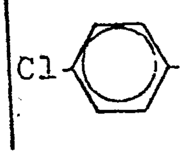 | $CH_3O-$ | 130 - 132 |

| Beispiel Nr. | $R_1$ | $R_2$ | $R_3$ | Fp. °C n (Brechungsindex) |
|---|---|---|---|---|
| 61 | 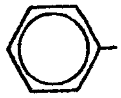 | $n-C_3H_7O-$ | $n-C_3H_7O-$ | $n_D^{25}=1,5056$ |
| 62 | 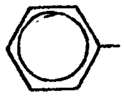 | $n-C_4H_9O-$ | $n-C_4H_9O-$ | $n_D^{25}=1,4988$ |
| 63 | 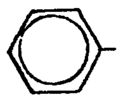 | $iso-C_3H_7O-$ | $iso-C_3H_7O-$ | 84-87 |
| 64 | 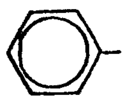 | $iso-C_4H_9O-$ | $iso-C_4H_9O-$ | $n_D^{20}=1,4990$ |
| 65 | 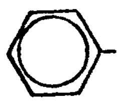 | $CH_3-$ | $CH_3O-$ | 78-87 |
| 66 | 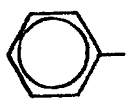 | 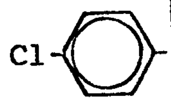 | $CH_3O-$ | 114-116 |
| 67 | 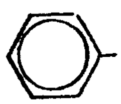 | $n-C_3H_7-$ | $C_2H_5O-$ | 80-82 |
| 68 | 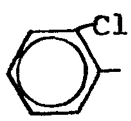 | $n-C_3H_7O-$ | $n-C_3H_7O-$ | $n_D^{30}=1,5075$ |
| 69 | 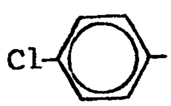 | $n-C_3H_7O-$ | $n-C_3H_7O-$ | 59-65 |
| 70 | 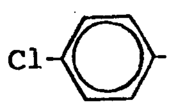 | $iso-C_3H_7O-$ | $iso-C_3H_7O-$ | 85-87 |
| 71 | 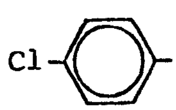 | $n-C_4H_9O-$ | $n-C_4H_9O-$ | 64-67 |
| 72 | 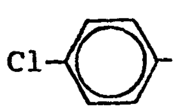 | $CH_3-$ | $CH_3O-$ | 114-116 |

| Beispiel Nr. | $R_1$ | $R_2$ | $R_3$ | Fp. °C n (Brechungs-index) |
|---|---|---|---|---|
| 73 | 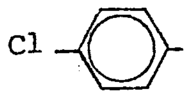 | $n-C_3H_7-$ | $C_2H_5O-$ | 100-102 |
| 74 | 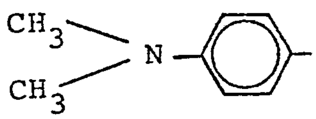 | $n-C_3H_7-$ | $C_2H_5O-$ | 103-106 |
| 75 | 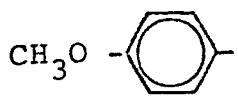 | $n-C_3H_7-$ | $C_2H_5O-$ | $n_D^{30}$=1,5172 |
| 76 | 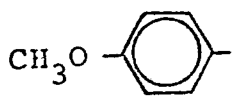 | 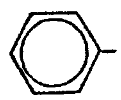 | $CH_3O-$ | |
| 77 | 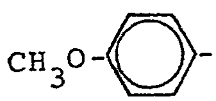 | $n-C_3H_7O-$ | $n-C_3H_7O-$ | 59-60 |
| 78 | 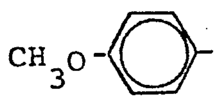 | $n-C_4H_9O-$ | $n-C_4H_9O-$ | $n_D^{25}$=1,5090 |
| 79 | 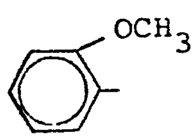 | $C_2H_5O-$ | $C_2H_5O-$ | 58-63 |
| 80 | 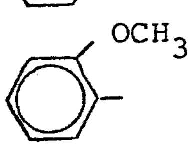 | $n-C_3H_7O-$ | $-C_3H_7O-$ | 53-55 |
| 81 | 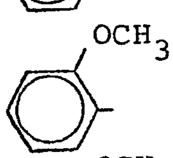 | iso-$C_3H_7O-$ | iso-$C_3H_7O-$ | 82-88 |
| 82 | 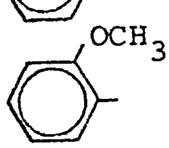 | $n-C_4H_9O-$ | $n-C_4H_9O-$ | $n_D^{30}$=1,5058 |
| 83 | 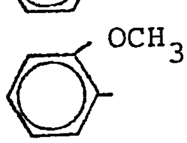 | iso-$C_4H_9O-$ | iso-$C_4H_9O-$ | $n_D^{30}$=1,5047 |
| 84 | 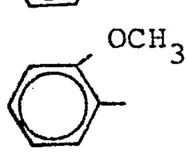 | $n-C_3H_7-$ | $C_2H_5O-$ | $n_D^{30}$=1,5172 |

| Beispiel Nr. | $R_1$ | $R_2$ | $R_3$ | Fp. °C n (Brechungsindex) |
|---|---|---|---|---|
| 85 | 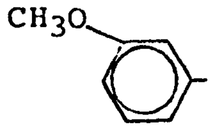 | $n-C_3H_7-$ | $C_2H_5O-$ | 77-80 |
| 86 | 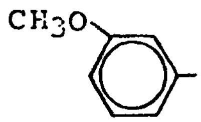 | 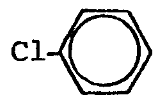 | $CH_3O-$ | 107-111 |
| 87 | 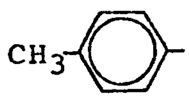 | $n-C_3H_7O-$ | $CH_3O-$ | 68-73 |
| 88 | 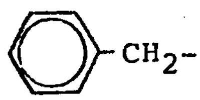 | $CH_3-$ | $CH_3-$ | 94 |
| 89 | 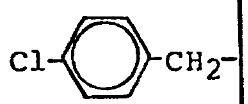 | $CH_3-$ | $CH_3-$ | |
| 90 | 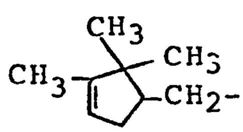 | $C_2H_5O-$ | $C_2H_5O-$ | $n_D^{25}=1,4775$ |
| 91 | $C_2H_5O-\overset{O}{\overset{\|}{C}}-$ | $CH_3-$ | $CH_3-$ | |
| 92 | $C_2H_5O-\overset{O}{\overset{\|}{C}}-$ | $CH_3-$ | 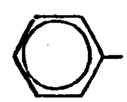 | |
| 93 | $CH_3O-\overset{O}{\overset{\|}{C}}-$ | $CH_3-$ | $CH_3O-$ | 114-118 |
| 94 | $C_2H_5O-\overset{O}{\overset{\|}{C}}-$ | $CH_3-$ | $C_2H_5O-$ | |
| 95 | $C_2H_5O-\overset{O}{\overset{\|}{C}}-$ | $C_2H_5O-$ | $C_2H_5O-$ | $n_D^{25}=1,4568$ |
| 96 | 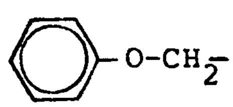 | $C_2H_5O-$ | $C_2H_5O-$ | |

| Beispiel Nr. | $R_1$ | $R_2$ | $R_3$ | Fp. °C n (Brechungsindex) |
|---|---|---|---|---|
| 97 | $CH_3O-CH_2-$ | $C_2H_5O-$ | $C_2H_5O-$ | |
| 98 | 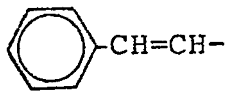 | $C_2H_5O-$ | $C_2H_5O-$ | |
| 99 | 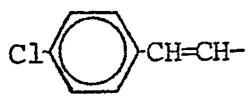 | $n-C_3H_7-$ | $C_2H_5O-$ | |
| 100 | 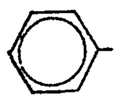 | $CH_3O-CH_2-$ | $CH_3O-$ | |
| 101 | 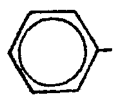 | 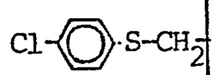 | $C_2H_5O-$ | |
| 102 | 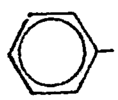 | $n-C_4H_9S-CH_2-$ | $C_2H_5O-$ | |
| 103 | 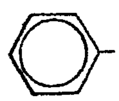 | 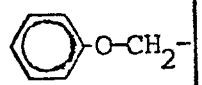 | $C_2H_5O-$ | |
| 104 | 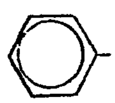 | 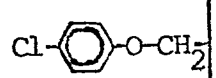 | $C_2H_5O-$ | |
| 105 | 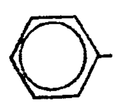 | 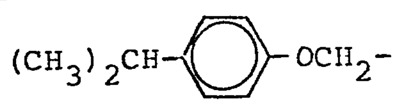 | $C_2H_5O-$ | |
| 106 | 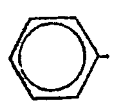 | $(C_2H_5O)_2CH-$ | $C_2H_5O-$ | $n_D^{30}$=1,4968 |
| 107 | 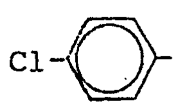 | $(C_2H_5O)_2CH-$ | $C_2H_5O-$ | $n_D^{22}$= 1,5139 |

| Beispiel Nr. | $R_1$ | $R_2$ | $R_3$ | Fp. °C n (Brechungsindex) |
|---|---|---|---|---|
| 108 | 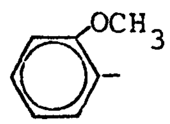 | $(C_2H_5O)_2CH-$ | $C_2H_5O-$ | $n_D^{30}$= 1,5071 |
| 109 | 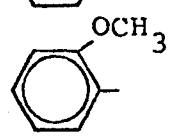 | $ClCH_2CH_2O-$ | $ClCH_2CH_2O-$ | |
| 110 | 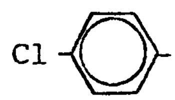 | $CH_3O-CH_2CH_2O-$ | $CH_3O-CH_2CH_2O-$ | |
| 111 | 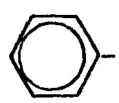 | $n-C_4H_9O-CH_2CH_2O-$ | $n-C_4H_9O-CH_2CH_2O-$ | $n_D^{30}$=1,4941 |
| 112 | 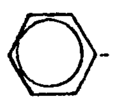 | $CH_3-$ | $C_2H_5S-CH_2CH_2O-$ | |
| 113 | 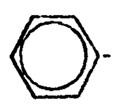 | $CH_3-$ | 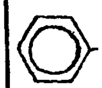$-O-CH_2CH_2O-$ | |
| 114 | 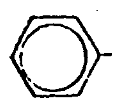 | $CH_3-$ | 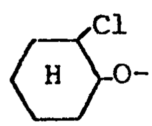 | |
| 115 | 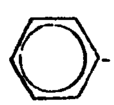 | $\genfrac{}{}{0pt}{}{CH_3}{CH_3}>N-CH_2CH_2O-$ | $\genfrac{}{}{0pt}{}{CH_3}{CH_3}>N-CH_2CH_2O-$ | |
| 116 | 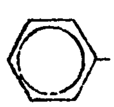 | $CH_2=CH-CH_2O-$ | $CH_2=CH-CH_2O-$ | |
| 117 | 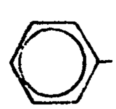 | $CH_3-$ | $CH_3-CH=CH-CH_2O-$ | |
| 118 | 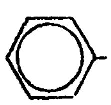 | $CH_3-$ | $Br-CH=CH-CH_2O-$ | |
| 119 | 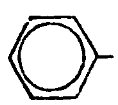 | $CH_3-$ | $C_2H_5-(O-CH_2CH_2)_2-O-$ | |

| Beispiel Nr. | $R_1$ | $R_2$ | $R_3$ | Fp. °C n (Brechungsindex) |
|---|---|---|---|---|
| 120 | Cl-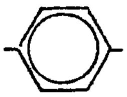 | $CH_3-$ | $CH\equiv C-CH_2O-$ | |
| 121 | $CH_3O$-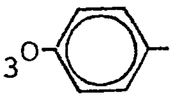 | $CH_3-$ | $CH\equiv C-CHO$<br>$CH_3$ | |
| 122 | $CH_3O$-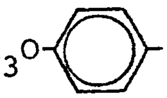 | $CH_3-$ | Phenyl<br>$CH\equiv C-CHO-$ | |
| 123 | 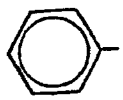 | $C_2H_5O-$ | Cl-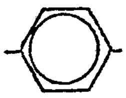-O- | |
| 124 | 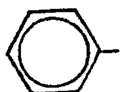 | $CH_3-$ | $CH_3-CHO-$<br>$CH_2Cl$ | |
| 125 | 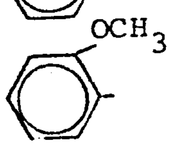 | $C_2H_5O-$ | $CH_3O-CH_2CH_2O-$ | |
| 126 | Cl-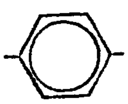 | $CH_3-$ | $(CH_3)_2N-$ | 143-146 |

B. Formulierungsbeispiele

Beispiel A

Ein Stäubemittel wird erhalten, indem man

10 Gewichtsteile Wirkstoff

und 90 Gewichtsteile Talkum als Inertstoff

mischt und in einer Schlagmühle zerkleinert.

Beispiel B

Ein in Wasser leicht dispergierbares benetzbares Pulver wird erhalten, indem man

25 Gewichtsteile Wirkstoff

64 Gewichtsteile kaolinhaltigen Quarz als Inertstoff

10 Gewichtsteile ligninsulfonsaures Kalium

und 1 Gewichtsteil oleoylmethyltaurinsaures Natrium als Netz- und Dispergiermittel

mischt und in einer Stiftmühle mahlt.

Beispiel C

Ein in Wasser leicht dispergierbares Dispersionskonzentrat wird erhalten, indem man

20 Gewichtsteile Wirkstoff mit

6 Gewichtsteilen Nonylphenolpolyglykoläther (10AeO)

3 Gewichtsteilen Isotridecanolpolyglykoläther (8 AeO)

und 71 Gewichtsteilen paraffinischem Mineralöl (Siedebereich 255 bis >377°C/Normaldruck)

mischt und in einer Reibkugelmühle auf eine Feinheit von unter 5 Mikron vermahlt.

Beispiel D

Ein emulgierbares Konzentrat wird erhalten aus

| | | |
|---|---|---|
| | 15 Gewichtsteilen | Wirkstoff |
| | 75 Gewichtsteilen | Cyclohexanon als Lösungsmittel |
| und | 10 Gewichtsteilen | oxäthyliertes Nonylphenol (10 AeO) als Emulgator. |

## C. Biologische Beispiele

### Beispiel I

Weizenpflanzen wurden im 3-Blattstadium mit Konidien des Weizenmehltaus (Erysiphe graminis) stark inokuliert und in einem Gewächshaus bei 20°C und einer relativen Luftfeuchte von 90 - 95 % aufgestellt. 3 Tage nach Inokulation wurden die Pflanzen mit den in Tabelle I aufgeführten Verbindungen in den Wirkstoffkonzentrationen von 500, 250, und 125 mg/Liter Spritzbrühe tropfnaß gespritzt. Als Vergleichsverbindung wurde Maneb eingesetzt. Nach einer Inkubationszeit von 10 Tagen wurden die Pflanzen auf Befall mit Weizenmehltau untersucht. Der Befallsgrad wurde ausgedrückt in % befallener Blattfläche bezogen auf unbehandelte, infizierte Kontrollpflanzen (= 100 % Befall). Das Ergebnis ist in der Tabelle I zusammengefaßt.

Tabelle I

| Verbindung gemäß Beispiel | mit Weizenmehltau befallene Blattfläche in % bei mg Wirkstoff/Liter Spritzbrühe | | | | |
|---|---|---|---|---|---|
| | 500 | 250 | 125 | 60 | 30 |
| 47 | 0 | 0 | 0 | | |
| 48 | 0 | 0 | 0-3 | | |
| 33 | 0 | 0 | 3-5 | | |
| 41 | 0 | 0 | 0-5 | | |
| 29 | 0 | 0 | 0 | | |
| 36 | 0 | 0 | 0-3 | | |
| 43 | 0 | 0 | 5 | | |

| Verbindung gemäß Beispiel | mit Weizenmehltau befallene Blattfläche in % bei mg Wirkstoff/Liter Spritzbrühe | | | | |
|---|---|---|---|---|---|
| | 500 | 250 | 125 | 60 | 30 |
| 32 | 0 | 0 | 3-5 | | |
| 2 | 0 | 0 | 0 | | |
| 5 | 0 | 0 | 0 | | |
| 61 | 0 | 0 | 0 | 0 | 0 |
| 62 | 0 | 0 | 0 | 0 | 0 |
| 63 | 0 | 0 | 0 | 0-3 | 3-5 |
| 64 | 0 | 0 | 0 | 0 | 0-3 |
| 65 | 0 | 0 | 0 | 0-3 | 3-5 |
| 67 | 0 | 0 | 0 | 0-3 | 3-5 |
| 68 | 0 | 0 | 0 | 0 | 0 |
| 69 | 0 | 0 | 0 | 0-3 | 3-5 |
| 72 | 0 | 0 | 0 | 0-3 | 3 |
| 77 | 0 | 0 | 0 | 0 | 0-3 |
| 78 | 0 | 0 | 0 | 0 | 0-3 |
| 79 | 0 | 0 | 0 | 0 | 0 |
| 80 | 0 | 0 | 0 | 0 | 0 |
| 81 | 0 | 0 | 0 | 0-3 | 3-5 |
| 82 | 0 | 0 | 0 | 0 | 0 |
| 83 | 0 | 0 | 0 | 0 | 0 |
| 84 | 0 | 0 | 0 | 0 | 0 |
| 85 | 0 | 0 | 0 | 0 | 0 |
| 87 | 0 | 0 | 0 | 0 | 0-3 |
| Maneb* | 5 | 10 | 15 | 100 | 100 |
| unbeh. infiz. Pflanzen | 100 | | | | |

*) Maneb = Mangan(II)-(N,N'-äthylen-bis-(dithiocarbamat))

Beispiel II

Gurkenpflanzen (Sorte Delikateß) wurden im 2-Blattstadium mit einer Konidiensuspension von Gurkenmehltau (Erysiphe cichoracearum) stark inokuliert. Nach einer Antrocknungszeit der Sporensuspension von 30 Minuten wurden die Pflanzen in einem Gewächshaus bei 22°C und 90 % relativer Luftfeuchte aufgestellt. 3 Tage nach Infektion wurden die Pflanzen mit den in Tabelle 2 genannten Verbindungen und Wirkstoffkonzentrationen tropfnaß gespritzt. Als Vergleichsverbindung wurde Maneb eingesetzt. Nach 10 Tagen erfolgte die Bonitur. Der Befallsgrad wurde ausgedrückt in % befallener Blattfläche, bezogen auf unbehandelte, infizierte Kontrollpflanzen (= 100 % Befall). Das Ergebnis ist in der Tabelle II zusammengefaßt.

Tabelle II

| Verbindung gemäß Beispiel | mit Gurkenmehltau befallene Blattfläche in % bei mg Wirkstoff/Liter Spritzbrühe | | | | |
|---|---|---|---|---|---|
| | 500 | 250 | 125 | 60 | 30 |
| 3 | 0 | 0 | 0-3 | | |
| 48 | 0 | 0 | 5 | | |
| 33 | 0 | 0 | 0 | | |
| 29 | 0 | 0 | 5 | | |
| 51 | 0 | 0 | 3-5 | | |
| 32 | 0 | 5 | 15 | | |
| 2 | 0 | 0 | 0 | | |
| 5 | 0 | 0 | 5 | | |
| 4 | 0 | 5-10 | 15-25 | | |
| 30 | 0 | 0 | 5 | | |
| 61 | 0 | 0 | 0 | 0 | 0 |
| 62 | 0 | 0 | 0 | 0 | 0 |

| Verbindung gemäß Beispiel | mit Gurkenmehltau befallene Blattfläche in % bei mg Wirkstoff/Liter Spritzbrühe 500 | 250 | 125 | 60 | 30 |
|---|---|---|---|---|---|
| 63 | 0 | 0 | 0 | 0 | 0-3 |
| 64 | 0 | 0 | 0 | 0 | 0 |
| 67 | 0 | 0 | 0 | 0 | 0 |
| 68 | 0 | 0 | 0 | 0 | 0 |
| 69 | 0 | 0 | 0 | 0 | 0-3 |
| 72 | 0 | 0 | 0 | 0-3 | 3-5 |
| 77 | 0 | 0 | 0 | 0 | 0 |
| 78 | 0 | 0 | 0 | 0 | 0 |
| 79 | 0 | 0 | 0 | 0 | 0-3 |
| 80 | 0 | 0 | 0 | 0 | 0 |
| 81 | 0 | 0 | 0 | 0 | 0-3 |
| 82 | 0 | 0 | 0 | 0 | 0 |
| 83 | 0 | 0 | 0 | 0 | 0 |
| 75 | 0 | 0 | 0 | 0 | 0-3 |
| 84 | 0 | 0 | 0 | 0 | 0 |
| 85 | 0 | 0 | 0 | 0 | 0 |
| 87 | 0 | 0 | 0 | 0 | 0-3 |
| Maneb*) | 15 | 25 | 35 | 100 | 100 |
| unbeh. infiz. Pflanzen | 100 | | | | |

*) vgl. Tabelle I

Beispiel III

Gurkenpflanzen (Sorte Delikateß) wurden im 2-Blattstadium mit einer Konidiensuspension eines Benomyl-resistenten Gurkenmehltaustammes (Erysiphe cichoracearum) stark inokuliert. Nach einer Antrocknungszeit der Sporensuspension von 30 Minuten wurden die Pflanzen in einem Gewächshaus bei 22°C und 90 % relativer Luftfeuchte aufgestellt. 3 Tage nach Infektion wurden die Pflanzen mit den in Tabelle III genannten Verbindungen und Wirkstoffkonzentrationen tropfnaß gespritzt. Als Vergleichsverbindungen wurden Maneb und Benomyl eingesetzt. Nach 10 Tagen erfolgte die Bonitur. Der Befallsgrad wurde ausgedrückt in % befallener Blattfläche, bezogen auf unbehandelte, infizierte Kontrollpflanzen (= 100 % Befall). Das Ergebnis ist in der Tabelle III zusammengefaßt.

Tabelle III

| Verbindung gemäß Beispiel | mit Gurkenmehltau (Benomyl-resistenter Stamm) befallene Blattfläche in % bei mg Wirkstoff/Liter Spritzbrühe | | | | |
|---|---|---|---|---|---|
| | 500 | 250 | 125 | 60 | 30 |
| 3 | 0 | 0 | 3-5 | | |
| 48 | 0 | 0 | 5-10 | | |
| 33 | 0 | 0 | 0 | | |
| 29 | 0 | 3-5 | 5-10 | | |
| 4 | 0 | 5-10 | 15-25 | | |
| 30 | 0 | 0-3 | 5 | | |
| 61 | 0 | 0 | 0 | 0 | 0 |
| 62 | 0 | 0 | 0 | 0 | 0 |
| 63 | 0 | 0 | 0 | 0 | 3 |
| 64 | 0 | 0 | 0 | 0 | 0 |
| 67 | 0 | 0 | 0 | 0 | 0-3 |

| Verbindung gemäß Beispiel | mit Gurkenmehltau (Benomyl-resistenter Stamm) befallene Blattfläche in % bei mg Wirkstoff/Liter Spritzbrühe 500 | 250 | 125 | 60 | 30 |
|---|---|---|---|---|---|
| 68 | 0 | 0 | 0 | 0 | 0 |
| 69 | 0 | 0 | 0 | 0-3 | 3 |
| 77 | 0 | 0 | 0 | 0 | 0 |
| 78 | 0 | 0 | 0 | 0 | 0 |
| 79 | 0 | 0 | 0 | 0 | 0 |
| 80 | 0 | 0 | 0 | 0 | 0 |
| 81 | 0 | 0 | 0 | 0 | 3 |
| 82 | 0 | 0 | 0 | 0 | 0 |
| 83 | 0 | 0 | 0 | 0 | 0 |
| 75 | 0 | 0 | 0 | 0 | 3 |
| 84 | 0 | 0 | 0 | 0 | 0 |
| 85 | 0 | 0 | 0 | 0 | 0-3 |
| 87 | 0 | 0 | 0 | 0 | 3 |
| Maneb*) | 15 | 25 | 35 | 100 | 100 |
| Benomyl**) | 35 | 60 | 100 | | |
| unbeh. infiz. Pflanzen | 100 | | | | |

*) vgl. Tabelle I; **) Benomyl = 1-(N-Butylcarbamoyl)-2-(methoxy-carboxamido)-benzimidazol

Beispiel IV

Gerstenpflanzen wurden im 3-Blattstaium mit Konidien des Gerstenmehltaus (Erysiphe graminis sp. hordei) stark inokuliert und in einem Gewächshaus bei 20°C und einer relativen Luftfeuchte von 90-95 % aufgestellt. 3 Tage nach Inokulation wurden die Pflanzen mit den in Tabelle IV aufgeführten Verbindungen in den Wirkstoffkonzentrationen von 500, 250 und 125 mg/Liter Spritzbrühe tropfnaß gespritzt. Als Vergleichsverbindung wurde Maneb eingesetzt. Nach einer Inkubationszeit von 10 Tagen wurden die Pflanzen auf Befall mit Gerstenmehltau untersucht. Der Befallsgrad wurde ausgedrückt in % befallener Blattfläche, bezogen auf unbehandelte, infizierte Kontrollpflanzen (= 100 % Befall). Das Ergebnis ist in der Tabelle IV zusammengefaßt.

Tabelle IV
==========

| Verbindung gemäß Beispiel | mit Gerstenmehltau befallene Blattfläche in % bei mg Wirkstoff/Liter Spritzbrühe | | | | |
|---|---|---|---|---|---|
| | 500 | 250 | 125 | 60 | 30 |
| 3 | 0 | 0 | 10-15 | | |
| 47 | 0 | 5 | 10-15 | | |
| 33 | 0 | 5 | 10-15 | | |
| 29 | 0 | 5 | 10-15 | | |
| 51 | 0 | 5 | 10-15 | | |
| 30 | 0 | 0 | 5-10 | | |
| 61 | 0 | 0 | 0 | 0 | 0 |
| 62 | 0 | 0 | 0 | 0 | 0 |
| 64 | 0 | 0 | 0 | 0 | 0-3 |
| 65 | 0 | 0 | 0 | 0 | 0-3 |

| Verbindung gemäß Beispiel | mit Gerstenmehltau befallene Blattfläche in % bei mg Wirkstoff/Liter Spritzbrühe 500 | 250 | 125 | 60 | 30 |
|---|---|---|---|---|---|
| 67 | 0 | 0 | 0 | 0 | 0 |
| 68 | 0 | 0 | 0 | 0 | 0 |
| 69 | 0 | 0 | 0 | 0-3 | 3 |
| 72 | 0 | 0 | 0 | 0-3 | 3 |
| 73 | 0 | 0 | 0 | 0 | 0-3 |
| 77 | 0 | 0 | 0 | 0 | 0 |
| 78 | 0 | 0 | 0 | 0 | 0-3 |
| 79 | 0 | 0 | 0 | 0 | 0 |
| 80 | 0 | 0 | 0 | 0 | 0 |
| 81 | 0 | 0 | 0 | 0-3 | 3-5 |
| 82 | 0 | 0 | 0 | 0 | 0 |
| 83 | 0 | 0 | 0 | 0 | 0-3 |
| 75 | 0 | 0 | 0 | 0-3 | 3-5 |
| 84 | 0 | 0 | 0 | 0 | 0 |
| 85 | 0 | 0 | 0 | 0 | 0 |
| 87 | 0 | 0 | 0 | 0 | 0 |
| Maneb*) | 25 | 35 | 60 | 100 | 100 |
| unbeh. infiz. Pflanzen | 100 | | | | |

*) vgl. Tabelle I

Beispiel V

Weinpflanzen, die aus Stecklingen der Plasmopara-anfälligen Sorte Müller-Thurgau gezogen waren, wurden im 4-Blattstadium mit wäßrigen Suspensionen der in Tabelle V aufgeführten Verbindungen und Wirkstoffkonzentrationen tropfnaß gespritzt.

Nach dem Antrocknen des Spritzbelages wurden die Pflanzen mit einer Zoosporangiensuspension von Plasmopara viticola inokuliert und tropfnaß in eine Klimakammer bei einer Temperatur von 20°C und einer relativen Luftfeuchtigkeit von 100 % gestellt. Nach 24 Stunden wurden die infizierten Pflanzen der Klimakammer entnommen und in ein Gewächshaus mit einer Temperatur von 23°C und einer Luftfeuchtigkeit von ca. 80-90 % gebracht.

Nach einer Inkubationszeit von 7 Tagen wurden die Pflanzen angefeuchtet, über Nacht in die Klimakammer gestellt und die Krankheit zum Ausbruch gebracht. Anschließend erfolgte die Befallsauswertung. Der Befallsgrad wurde in % befallender Blattfläche im Vergleich zu den unbehandelten, infizierten Kontrollpflanzen ausgedrückt und ist in Tabelle V wiedergegeben.

Tabelle V

| Verbindung gemäß Beispiel | % Plasmopara-Befall bei mg Wirkstoff/ Liter Spritzbrühe | | | |
|---|---|---|---|---|
| | 500 | 250 | 125 | |
| 3 | 0 | 0-3 | 30 | |
| 30 | 0 | 0 | 3-5 | |
| 33 | 0 | 0 | 3-5 | |
| 59 | 0 | 0 | 5-10 | |
| 24 | 0 | 0 | 5-10 | |
| 29 | 0 | 0 | 3-5 | |
| 64 | 0 | 0 | 3-5 | |
| 65 | 0 | 0 | 0 | |
| unbeh. infiz. Pflanzen | 100 | | | |

Patentansprüche:

1. Verbindungen der allgemeinen Formel I,

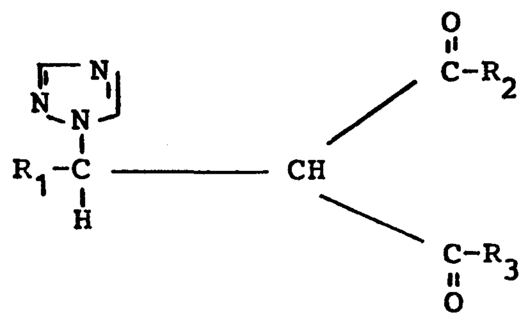

worin

$R_1$ $(C_1-C_{12})$-Alkyl, das gegebenenfalls zusätzlich vorzugsweise durch Halogen, Phenyl, das ein- bis zweifach durch Halogen, $(C_1-C_4)$-Alkoxy oder $(C_1-C_4)$-Alkyl substituiert sein kann, $(C_1-C_4)$-Alkoxy oder Phenoxy, welches ein- bis zweifach durch Halogen, $(C_1-C_4)$-Alkoxy oder $(C_1-C_4)$-Alkyl substituiert sein kann, substituiert ist, $(C_2-C_5)$-Alkenyl, das gegebenenfalls zusätzlich durch Phenyl, das ein- bis zweifach durch Halogen oder $(C_1-C_4)$-Alkoxy substituiert sein kann, substituiert ist, Cycloalkyl, vorzugsweise mit 5 oder 6 C-Atomen, oder substituiertes Cycloalkyl mit vorzugsweise insgesamt 5 bis 9 C-Atomen, insbesondere durch Alkylreste substituiertes Cycloalkyl, Cycloalkenyl, vorzugsweise mit 5 oder 6 C-Atomen, oder substituiertes Cycloalkenyl mit vorzugsweise insgesamt 5 bis 9 C-Atomen, insbesondere durch Alkylreste substituiertes Cycloalkenyl, Phenyl oder substituiertes Phenyl, das insbesondere ein- bis dreifach durch Alkyl, vorzugsweise $(C_1-C_{12})$-Alkyl, Halogen, $(C_1-C_5)$-Alkoxy, Hydroxy, Nitro, Dialkylamino mit vorzugsweise $(C_1-C_6)$-Alkylgruppen, oder den Methylendioxy-Rest substituiert ist, weiterhin Furanyl, Thienyl, Pyridyl

oder $(C_1-C_4)$-Alkoxycarbonyl, und

$R_2$, $R_3$, die gleich oder verschieden sein können, $(C_1-C_{12})$-Alkyl, das gegebenenfalls zusätzlich vorzugsweise durch Halogen, insbesondere ein- oder zweifach, und/oder ein- oder zweifach durch $(C_1-C_6)$-Alkoxy, ferner durch $(C_1-C_4)$-Alkylthio, Phenylthio, welches ein- oder zweifach durch Halogen oder $(C_1-C_4)$-Alkyl substituiert sein kann, $(C_1-C_4)$-Alkylamino, Di-$(C_1-C_4)$-alkylamino, Phenyl oder Phenoxy, welches ein- oder zweifach durch Halogen, $(C_1-C_4)$-Alkyl oder $(C_1-C_3)$-Alkoxy substituiert sein kann, substituiert ist, oder Cycloalkyl, vorzugsweise mit 5 oder 6 C-Atomen, oder substituiertes Cycloalkyl mit vorzugsweise insgesamt 5 bis 9 C-Atomen, insbesondere durch Alkylreste substituiertes Cycloalkyl, Phenyl oder substituiertes Phenyl, das insbesondere ein- bis dreifach durch Alkyl, vorzugsweise $(C_1-C_{12})$-Alkyl, Halogen, $(C_1-C_5)$-Alkoxy oder Hydroxy substituiert ist, $(C_1-C_{12})$-Alkoxy, das gegebenenfalls zusätzlich durch 1 bis 6, vorzugsweise 1 oder 2 Halogenatome und/oder $(C_1-C_6)$-Alkoxy, $(C_1-C_4)$-Alkylthio, $(C_1-C_6)$-Alkoxy-$(C_2-C_6)$-alkoxy, Halogen-$(C_1-C_2)$-alkoxy, Methoxy-äthoxy-äthoxy, $(C_1-C_4)$-Alkylamino, Di-$(C_1-C_4)$-alkylamino, Phenyl, das ein- oder zweifach durch Halogen, $(C_1-C_4)$-Alkyl, $(C_1-C_3)$-Alkoxy oder N-$(C_1-C_4)$-Alkyl-anilino substituiert sein kann, Oxiranyl oder Phenoxy, das ein oder zweifach durch Halogen oder $(C_1-C_4)$-Alkyl substituiert sein kann, substituiert ist, oder $(C_5-C_6)$-Cycloalkoxy, das gegebenenfalls durch Halogen oder Methyl substituiert ist, $(C_3-C_6)$-Alkenyloxy, Halogen-$(C_3-C_6)$-alkenyloxy, $(C_5-C_6)$-Cycloalkenyloxy, $(C_3-C_4)$-Alkinyloxy, das gegebenenfalls ein- oder zweifach durch $(C_1-C_6)$-Alkyl, Phenyl, Halogen oder $(C_1-C_2)$-Alkoxy substituiert ist, oder Phenoxy, das gegebenenfalls ein- bis dreifach durch $(C_1-C_4)$-Alkyl, $(C_1-C_4)$-

Alkoxy, Halogen, Nitro oder Trifluormethyl substituiert ist, oder $(C_1-C_4)$-Alkylamino, Di-$(C_1-C_4)$-alkylamino, Anilino, das gegebenenfalls am Benzolring ein- oder zweifach durch $(C_1-C_4)$-Alkyl, Halogen, $(C_1-C_3)$-Alkoxy oder am Anilin-N-atom durch eine $(C_1-C_4)$-Alkylgruppe substituiert ist, bedeuten.

2. Verfahren zur Herstellung von Verbindungen der Formel I gemäß Anspruch 1, dadurch gekennzeichnet, daß man Verbindungen der allgemeinen Formel II,

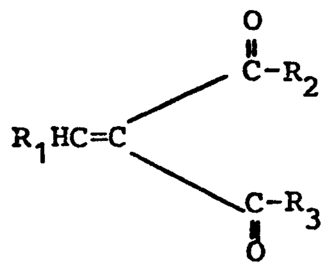

(II)

worin $R_1$, $R_2$ und $R_3$ die Bedeutungen wie in Formel I haben, mit 1, 2, 4-Triazol umsetzt.

3. Verfahren nach Anspruch 2, dadurch gekennzeichnet, daß man die Umsetzung in Gegenwart eines basischen Katalysators, vorzugsweise eines tertiären Amins durchführt.

4. Verfahren nach Ansprüchen 2 und 3, dadurch gekennzeichnet, daß man die Umsetzung in einem inerten Lösungsmittel durchführt.

5. Schädlingsbekämpfungsmittel, gekennzeichnet durch einen Gehalt an einer Verbindung der Formel I gemäß Anspruch 1 als Wirkstoff.

6. Schädlingsbekämpfungsmittel, gekennzeichnet durch einen Gehalt von 2 bis 95 Gew.-%, vorzugsweise 10 bis 90 Gew.-% an einer Verbindung der Formel I gemäß Anspruch 1 als Wirkstoff sowie üblichen Formulierungshilfsmitteln.

7. Verwendung von Verbindungen der Formel I gemäß Ansprüchen 1, 5 und 6 zur Schädlingsbekämpfung im Pflanzenschutz.

8. Verfahren zur Bekämpfung von Schadpilzen, dadurch gekennzeichnet, daß man die von ihnen befallenen Flächen und/oder Pflanzen und/oder Substrate mit wirksamen Mengen von Wirkstoffen der Formel I gemäß Ansprüchen 1, 5 und 6 in Kontakt bringt.

Patentansprüche für Österreich:

1. Verfahren zur Herstellung von Verbindungen der Formel I,

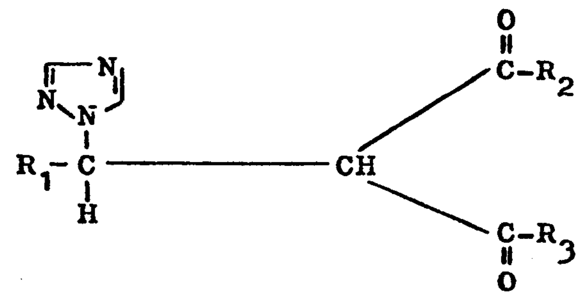

worin

$R_1$ $(C_1-C_{12})$-Alkyl, das gegebenenfalls zusätzlich vorzugsweise durch Halogen, Phenyl, das ein- bis zweifach durch Halogen, $(C_1-C_4)$-Alkoxy oder $(C_1-C_4)$-Alkyl substituiert sein kann, $(C_1-C_4)$-Alkoxy oder Phenoxy, welches ein- bis zweifach durch Halogen, $(C_1-C_4)$-Alkoxy oder $(C_1-C_4)$-Alkyl substituiert sein kann, substituiert ist, $(C_2-C_5)$-Alkenyl, das gegebenenfalls zusätzlich durch Phenyl, das ein- bis zweifach durch Halogen oder $(C_1-C_4)$-Alkoxy substituiert sein kann, substituiert ist, Cycloalkyl, vorzugsweise mit 5 oder 6 C-Atomen, oder substituiertes Cycloalkyl mit vorzugsweise insgesamt 5 bis 9 C-Atomen, insbesondere durch Alkylreste substituiertes Cycloalkyl, Cycloalkenyl, vorzugsweise mit 5 oder 6 C-Atomen, oder substituiertes Cycloalkenyl mit vorzugsweise insgesamt 5 bis 9 C-Atomen, insbesondere durch Alkylreste substituiertes Cycloalkenyl, Phenyl oder substituiertes Phenyl, das insbesondere ein- bis dreifach durch Alkyl, vorzugsweise $(C_1-C_{12})$-Alkyl, Halogen, $(C_1-C_5)$-Alkoxy, Hydroxy, Nitro, Dialkylamino mit vorzugsweise $(C_1-C_6)$-Alkyl-

gruppen, oder den Methylendioxy-Rest substituiert ist, weiterhin Furanyl, Thienyl, Pyridyl oder ($C_1$-$C_4$)-Alkoxycarbonyl, und

$R_2$,$R_3$, die gleich oder verschieden sein können, ($C_1$-$C_{12}$)-Alkyl, das gegebenenfalls zusätzlich vorzugsweise durch Halogen, insbesondere ein- oder zweifach, und/oder ein- oder zweifach durch ($C_1$-$C_6$)-Alkoxy, ferner durch ($C_1$-$C_4$)-Alkylthio, Phenylthio, welches ein- oder zweifach durch Halogen oder ($C_1$-$C_4$)-Alkyl substituiert sein kann, ($C_1$-$C_4$)-Alkylamino, Di-($C_1$-$C_4$)-alkylamino, Phenyl oder Phenoxy, welches ein- oder zweifach durch Halogen, ($C_1$-$C_4$)-Alkyl oder ($C_1$-$C_3$)-Alkoxy substituiert sein kann, substituiert ist, oder Cycloalkyl, vorzugsweise mit 5 oder 6 C-Atomen, oder substituiertes Cycloalkyl mit vorzugsweise insgesamt 5 bis 9 C-Atomen, insbesondere durch Alkylreste substituiertes Cycloalkyl, Phenyl oder substituiertes Phenyl, das insbesondere ein- bis dreifach durch Alkyl, vorzugsweise ($C_1$-$C_{12}$)-Alkyl, Halogen, ($C_1$-$C_5$)-Alkoxy oder Hydroxy substituiert ist, ($C_1$-$C_{12}$)-Alkoxy, das gegebenenfalls zusätzlich durch 1 bis 6, vorzugsweise 1 oder 2 Halogenatome und/oder ($C_1$-$C_6$)-Alkoxy, ($C_1$-$C_4$)-Alkylthio, ($C_1$-$C_6$)-Alkoxy-($C_2$-$C_6$)-alkoxy, Halogen-($C_1$-$C_2$)-alkoxy, Methoxy-äthoxy-äthoxy, ($C_1$-$C_4$)-Alkylamino, Di-($C_1$-$C_4$)-alkylamino, Phenyl, das ein- oder zweifach durch Halogen, ($C_1$-$C_4$)-Alkyl, ($C_1$-$C_3$)-Alkoxy oder N-($C_1$-$C_4$)-Alkyl-anilino substituiert sein kann, Oxiranyl oder Phenoxy, das ein oder zweifach durch Halogen oder ($C_1$-$C_4$)-Alkyl substituiert sein kann, substituiert ist, oder ($C_5$-$C_6$)-Cycloalkoxy, das gegebenenfalls durch Halogen oder Methyl substituiert ist, ($C_3$-$C_6$)-Alkenyloxy, Halogen-($C_3$-$C_6$)-alkenyloxy, ($C_5$-$C_6$)-Cycloalkenyloxy, ($C_3$-$C_4$)-Alkinyloxy, das gegebenenfalls ein- oder zweifach durch ($C_1$-$C_6$)-Alkyl, Phenyl, Halogen oder

($C_1$-$C_2$)-Alkoxy substituiert ist, oder Phenoxy, das gegebenenfalls ein- bis dreifach durch ($C_1$-$C_4$)-Alkyl, ($C_1$-$C_4$)-Alkoxy, Halogen, Nitro oder Trifluormethyl substituiert ist, oder ($C_1$-$C_4$)-Alkylamino, Di-($C_1$-$C_4$)-alkylamino, Anilino, das gegebenenfalls am Benzolring ein- oder zweifach durch ($C_1$-$C_4$)-Alkyl, Halogen, ($C_1$-$C_3$)-Alkoxy oder am Anilin-N-atom durch eine ($C_1$-$C_4$)-Alkylgruppe substituiert ist, bedeuten,dadurch gekennzeichnet, daß man Verbindungen der allgemeinen Formel II,

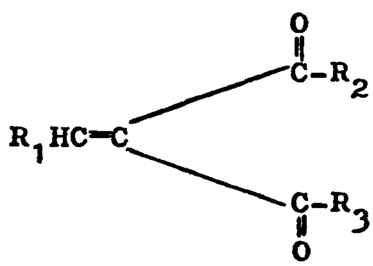

(II)

mit 1, 2, 4-Triazol umsetzt.

2. Verfahren nach Anspruch 1. dadurch gekennzeichnet, daß man die Umsetzung in Gegenwart eines basischen Katalysators, vorzugsweise eines tertiären Amins durchführt.

3. Verfahren nach Ansprüchen 1.und 2. dadurch gekennzeichnet, daß man die Umsetzung in einem inerten Lösungsmittel durchführt.

4. Schädlingsbekämpfungsmittel, gekennzeichnet durch einen Gehalt an einer Verbindung der Formel I als Wirkstoff.

5. Schädlingsbekämpfungsmittel, gekennzeichnet durch einen Gehalt von 2 bis 95 Gew.-%, vorzugsweise 10 bis 90 Gew.-% an einer Verbindung der Formel I als Wirkstoff sowie üblichen Formulierungshilfsmitteln.

6. Verwendung von Verbindungen der Formel I zur Schädlingsbekämpfung im Pflanzenschutz.

7. Verfahren zur Bekämpfung von Schadpilzen, dadurch gekennzeichnet, daß man die von ihnen befallenen Flächen und/oder Pflanzen und/oder Substrate mit wirksamen Mengen von Wirkstoffen der Formel I in Kontakt bringt.

0008458

Europäisches Patentamt

# EUROPÄISCHER RECHERCHENBERICHT

Nummer der Anmeldung

EP 79 10 3077

## EINSCHLÄGIGE DOKUMENTE

| Kategorie | Kennzeichnung des Dokuments mit Angabe, soweit erforderlich, der maßgeblichen Teile | betrifft Anspruch |
| --- | --- | --- |
| A | NL - A - 76 05615 (CIBA-GEIGY)<br>---- | |

KLASSIFIKATION DER ANMELDUNG (Int.Cl. 3)

C 07 D 249/08
409/06
405/06
401/06
A 01 N 43/64

RECHERCHIERTE SACHGEBIETE (Int. Cl. 3)

C 07 D 249/08
409/06
405/06
401/06

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
| --- | --- | --- |
| Den Haag | 22-11-1979 | CREMERS |